# EUROPEAN PATENT APPLICATION

(11) **EP 1 374 917 A1**
(43) Date of publication of application: **02.01.2004**
(21) Application number: 03076031.8
(22) Date of filing: 03.04.2003
(51) Int. Cl.: A61L 2/26, F16L 37/38, B65D 88/74

(54) **Method to carry out sanitization that is disinfection and/or sterilization and/or disinfestation of limited areas with difficult or foreclosured access**

(30) Priority: 04.04.2002 IT BA20020014
(71) Applicant: Caracciolo, Alessio Maria, 74020 Lama (TA) (IT); Caracciolo, Elisabetta, 74020 Lama (TA) (IT)
(72) Inventor: Caracciolo, Marcello, 74020 Lama (TA) (IT)
(74) Representative: Russo, Saverio, Dott. Ing.

(57) **Abstract**

The object of the invention is a method to carry out sanitization that is disinfection and/or sterilization and/or disinfestation of closed spaces with difficult or foreclosured access.

In particular, the method is to be applied to containers, railway wagons, air conditioning systems, by means of a fixed special valve 1 which is connected to a generator 2 of the sanitizing fluid, through a flexible sanitary cable 19.

The valve is provided with a check device permitting the introduction of sanitizing fluids and preventing dangerous gases to outflow from the space to be sanitified.

The method can be applied to decontaminate any type of space that is contaminated by infective pathogenic agents.

## Description

The invention deals with a method to carry out sanitization that is disinfection and/or sterilization and/or disinfestation of limited spaces with difficult or foreclosured access.

As it is well known, containers stored at the piers of ports, containing various kinds of goods, are often provided with lock devices, for example the twist-lock device, which are often sealed by the customs authorities before the containers leave a country, both when they are involved in export-import trade activities and as a common precautionary measure.

Therefore, said lock devices do not allow any sanitization or microbicidal treatments on the goods loaded inside the containers, said treatments being necessary when the goods come from questionable places; on the other hand, the containers are very often piled up and therefore they are not easily reachable to carry out said treatments even if it is possible to unlock the containers.

These problems also occur with other means of transport as, for example, railway wagons used for transporting passengers, goods, animals, inside which indoor spaces must be sanitized both before shipping and after their arrival at the destined places, after closing said spaces when they are empty (if they are used for transporting passengers or animals).

Disinfection and/or sanitization are also to be necessarily carried out on the ducts of air conditioning systems, for which reason they are completely disassembled from time to time.

In particular, if said systems are installed in hospitals, it is absolutely necessary to take said means of prevention to the diffusion of pathogenic agents.

Furthermore, disinfection is also indispensable on boxes into which waste from hospitals is gathered for elimination after being previously treated.

Said cardboard boxes include a plastic bag inside which waste is gathered and then treated by means of disinfectant liquids there into vaporized, so that disinfection is only carried out on the surface and pathogenic microbes are not thoroughly eliminated.

Many cases might be mentioned in which disinfection, sterilization and disinfestation are to be necessarily carried out on plants having ducts through which the air is conveyed, in particular on air conditioning systems.

This invention aims at providing a method by which sanitization is rapidly carried out on spaces with difficult access.

Another aim of the invention is to provide a method by which sanitization is rapidly carried out on said spaces without the intervention of any operators inside there and without allowing pathogenic microbes or gases dangerous both to the environment and to human beings to come out of said disinfected spaces.

The problem is resolved by the use of a device that permits the immediate introduction of vaporized disinfectants produced, for example, by means of aerosol appliances specific to all kinds of disinfection, or by means of nebulizers for insecticides or for any other fluids.

The numerous advantages of the invention include, in particular, the rapidity and effectiveness of the treatments carried out, especially when sanitization is to be performed (as it happens with containers) on spaces with difficult access for which it would otherwise be necessary the aid of many operators.

The invention is here described with the help of some examples for the application of the device and the means that allow the sanitizing treatment, which are illustrated in the enclosed drawings.

Fig. 1: a detail - shown in its section - of the valve for the introduction of the sanitizing fluid with fixed cap;

Fig. 2: a detail - shown in its section - of the valve with disassembled cap;

Fig. 3: a detail of the joint by which the flexible sanitary cable is introduced, said cable being connected to an aerosol generator, before the assembly, and a view from a X point;

Fig. 4: a detail of the valve after the introduction of the flexible sanitary cable;

Fig. 5: the method applied to a container;

Fig. 6: the method applied to piled up containers;

Fig. 7: the method applied to a railway wagon;

Fig. 8: the method applied for the disinfection of the cabin of a small boat;

Fig. 9: the method applied to the ducts of air conditioning systems.

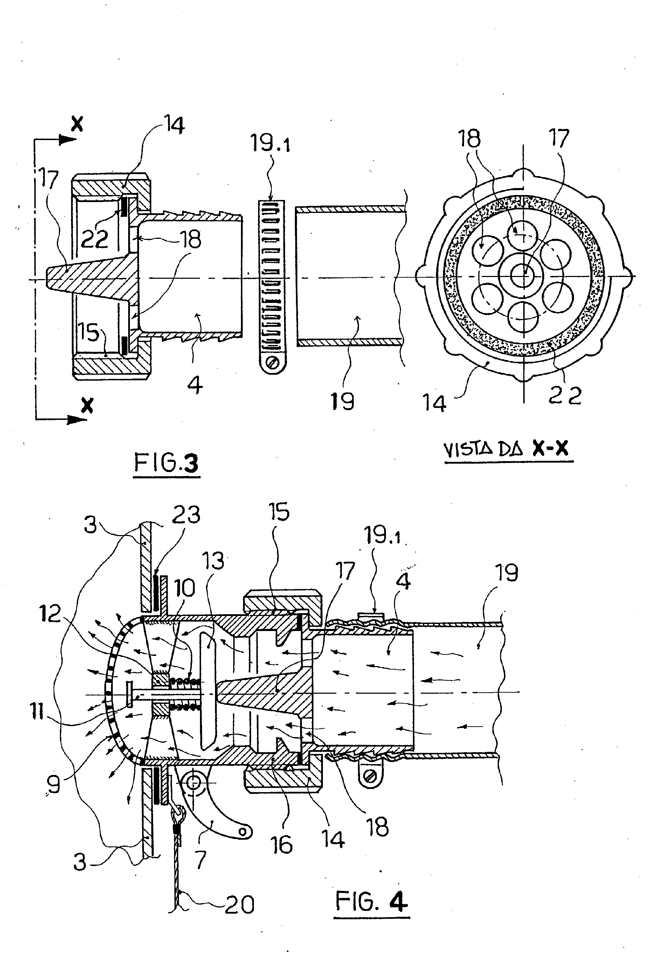

As it is illustrated in fig. 3 and fig. 4, the valve 1 for the application of the method according to the invention, is made in such a way as to allow a flexible sanitary cable 19 to be connected to the fluid which is being used to carry out sanitization with said flexible sanitary cable, by means of an aerosol generator or by means of nebulizers for insecticides or by using any other fluids.

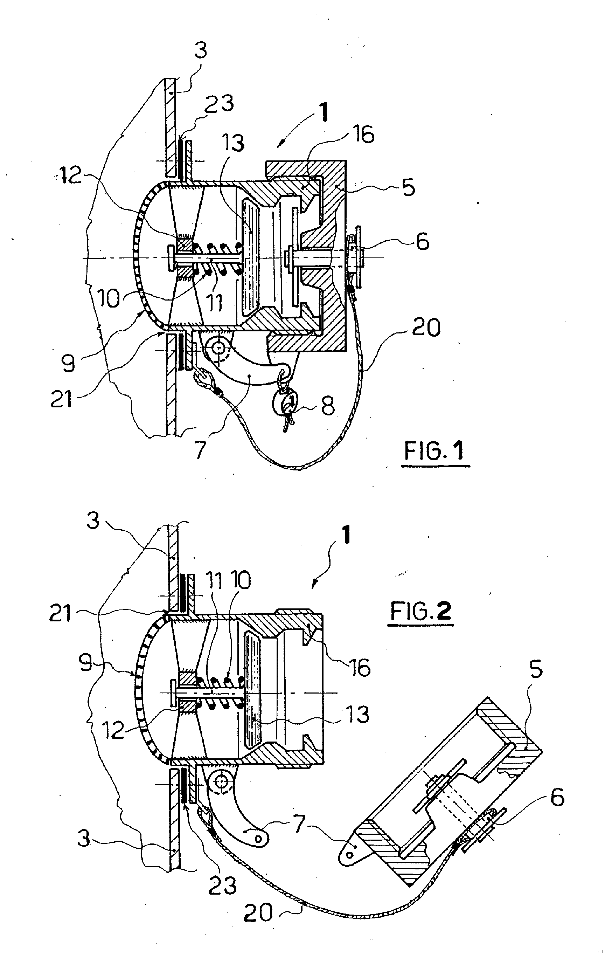

The valve 1 firmly joined to the metallic outer side 3 of the space to be sanitized, is of an irremovable type, for example by welding or by other permanent means 23 after inserting the extremity of the valve into a hole 21 of the metallic outer side, see fig. 1 and fig. 2.

The valve 1 is provided with a filtering net 9 and with a check valve 10 made of a shank 11 sliding into a cylindrical hole 12, said device being connected to a plate 13 which obstructs the outflow opening.

The valve and the extremity of the flexible sanitary cable may be connected by means of an element 4 provided with a joint band 14 which, in turn, is provided with a thread 15 by which said band is turned around the extremity 16 of the valve.

The joint element 4 is also provided with a pressure pin 17 by which the outflow opening obstructed by the plate 13 is opened, and with outflow holes 18.

The valve is closed by means of a cap 5, which may be firmly held in the closing position by means of a lock 6 and fastened through a device 7 allowing the seals 8 to be introduced there into. The cap 5 is also provided with a metallic cord 20 by which only, once opened, said cap is connected to the valve.

A particular feature of the valve is in the mechanical anti-violation characteristics of both the material and the manufacturing plan.

Numerous sanitizing treatments may be carried out by means of the valve so far described.

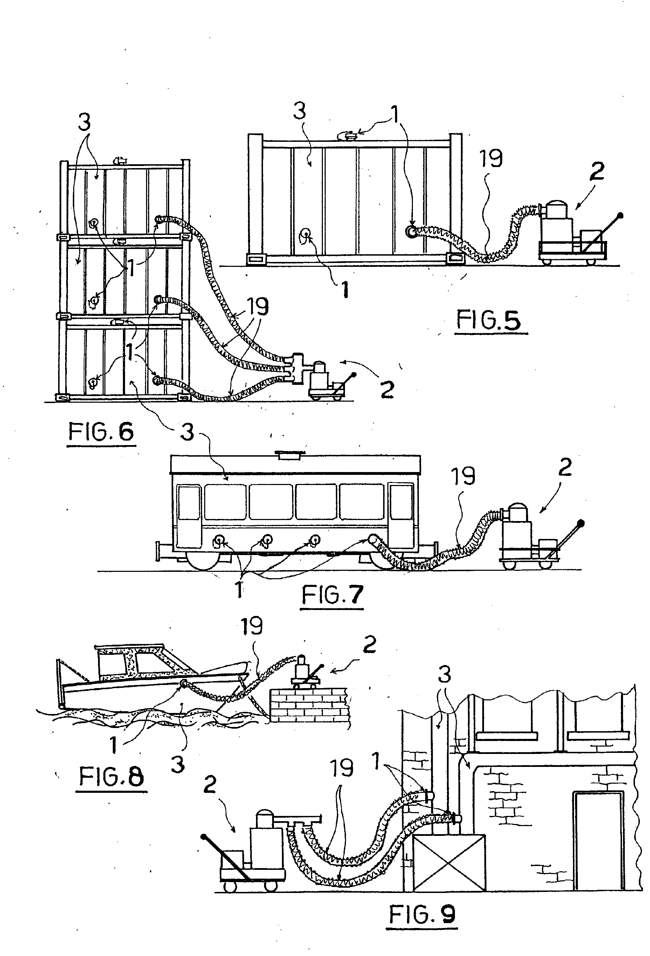

A first case of application is the disinfection of a container (fig. 5) stored in a container terminal, containing various kinds of goods, provided with the twist-lock device and with seals applied by the customs authorities before the container leaves a country, or in another case, the disinfection of containers piled up in order to save room (fig. 6) and not easily reachable (at about 6 metres from the ground).

In this case sanitization may not be performed if the container is closed and provided with irremovable seals, if it is loaded with goods which prevent the access there into, and if it is placed at a considerable height from the ground.

Sanitization is easily and rapidly carried out by applying the valve 1 (or several valves in the case of piled containers) to one side of the container, said valves being connected to the suitable aerosol generator 2 by means of one or several flexible sanitary cables 19.

Said sanitizing treatment may also be applied to any kind of railway wagons (fig. 7) or road vehicles, including refrigerator cars, and any vehicles used for transporting passengers, goods, animals which are to be sanitized both before the departure and after their arrival at the destined places, after airtightly closing them when they are empty, in case they are used for transporting passengers or animals.

Another case of application of the method under concern is the disinfection of the indoor space of a boat (fig. 8), or inside the ducts of an air conditioning system of a vessel (both a warship and a cargo boat) or of a submarine, in order to prevent contagions diseases from spreading.

At the present it is not possible to carry out disinfection on all the sections of air conditioning systems, and is not effective if it is carried out by means of commonly known procedures.

Disinfection is easily and rapidly carried out by applying the valve 1 (or several valves so as to cover the whole plant) directly on the air ducts, being helped by their hidden sides so as to avoid protrusions, then connecting the several valves to the suitable aerosol generator 2 by means of one or several flexible sanitary cables 19.

The method applied to the air conditioning system of a vessel or submarine may also be applied inside commercial centres, hospitals (fig. 9), as well as inside road vehicles, and so on.

A further case of application of the method according to the invention is the disinfection of a box used for transporting waste from hospitals there inside gathered, said waste being treated in order to prevent contagions diseases from spreading.

In these cases disinfection may not be carried out on all that is there inside gathered, because it would be otherwise necessary to empty the box and disinfect everything; moreover, the traditionally carried out treatment would involve the risk coming from dealing with dangerous materials.

The application of the valve 1 to the upper surface of the box would allow a rapid and easy treatment of the waste there inside gathered.

An aerosol generator suitable for the application of the method according to the invention should have the following characteristiques:
- being suitable to transform disinfectant, balsamic, antibiotic, anti-parasites substances from the static condition of liquid solutions to the dynamic condition of colloidal air-like substances, i.e. dry aerosol;
- being easily transported and handled, said generator also being moved by means of wheels;
- being suitable to be applied for the disinfection of large spaces or, for example, of more than one single container at the same time, since voluminous spaces may be treated by means of said generator;
- rapidity in carrying out disinfection, because said aerosol appliances have a very brief operation time;
- limited consumption of disinfectant;
- the aerosol emission is automatically interrupted and then again restarted.

Felmar 745®, would be a suitable aerosol appliance, having the following technical characteristiques:
Possibility to treat spaces with an up to 999 cm volume
Time necessary for the treatment: from 3 up to 50 minutes
Capacity: 1000 cc.
Weight: about 34 kg
Dimensions: 40x80x90 cm
Feeding tension: 220V 50Hz
Input: 250W

The method may also be applied by means of compressors placed inside the fluid circuit.

The method is particularly suitable for the sanitization of environments that are at infection risk, and in particular wards/divisions where there is a risk of infections.

## Claims

1. "Method to carry out sanitization that is disinfection and/or sterilization and/or disinfestation of limited spaces with difficult or floreclosured access", said spaces being containers, vehicles or railway wagons, ducts of air conditioning systems, infected spaces, packages for infected materials, and so on, **characterised by** the application of a fixed, irremovable valve 1 to the metallic and/or non-metallic air-tight sides of said spaces for the application of a flexible sanitary cable 19 through which a fluid suitable to carry out a specific sanitizing treatment is introduced.

2. Method as claimed in claim 1), **characterised by** the fact that the valve 1 has the following characteristiques when it is applied to particular means used for transporting goods, such as containers, railway wagons and road vehicles, including refrigerator cars, and in general to all means which carry goods there inside closed:
a) obstruction of the outflow opening by means of a cap provided with a lock-device;
b) possibility for a seal to be applied to the cap lock-device;
c) perfect tightness, i.e. suitability to prevent, by means of a check valve, any fluid outflow or inflow - even in case of fluids under pressure - when the valve is securely both closed and open by means of a suitable check device;
d) possibility to airtightly connect the extremity of a flexible sanitary cable by means of a thread, a pressure insertion, a joint, and so on, so that gases are not allowed to come out from the treated spaces while sanitization is performed;
e) mechanical anti-violation characteristiques of both the valve material and its manufacturing plan.

3. Method as claimed in claim 1), **characterised by** the fact that the fluid generator is placed outside the space to be treated and it does not require the intervention of any operators inside said space.

4. Method as claimed in claims 1) and 3), **characterised by** the fact the a suitable generator has at the same time the following characteristiques:
• being suitable to produce aerosol;
• being easily transported and handled by means of wheels, fastening and check valves;
• being suitable for the treatment of voluminous spaces;
• the cycle of emission has brief times of automatic intervention;
• limited consumption of disinfectant;
• the aerosol emission is automatically interrupted and then again restarted, if necessary.

5. Method as claimed in previous claims, **characterised by** the fact that it is possible to carry out sanitization on more than one single container at the same time - i.e. piled up containers - by means of flexible sanitary cables connected to a single aerosol generator.

6. Method as claimed in previous claims, **characterised by** the application of a compressor inside the fluid circuit.

7. Valve to perform the treatments as claimed in claim 1), **characterised by**:
• a fixed, irremovable connection to the metallic outer side of the space to be treated;
• a filtering section 9;
• a check valve 10 permitting the introduction of the sanitizing fluid and preventing dangerous gases to outflow from the space to be treated ;
• a cap which is firmly fixed to the valve by means of a lock-device;
• device by which the cap is sealed.

8. Method as claimed in claim 1) and valve as claimed in claim 7), **characterised by** the fact that the extremity of the flexible sanitary cable used for introducing the fluid is provided with a joint element having a pin 17 which mechanically unfastens, through pressure, the valve blocking device formed by the plate 13.

9. Method as claimed in previous claims **characterised by** the fact that the valve can permit only the application of the sanitization devices according to the invention, i.e. aerosol, compressor, etc.

10. Application of the method claimed in previous claims **characterised in** the sanitization of wards of infective diseases of hospitals, clinics, etc.
